# EUROPEAN PATENT SPECIFICATION

(11) **EP 3 698 814 B1**
(45) Date of publication and mention of the grant of the patent: **10.09.2025**
(21) Application number: 18868312.2
(22) Date of filing: 18.07.2018
(51) Int. Cl.: A61L 27/28, A61L 27/38, A61L 31/08, A61L 27/34, A61L 27/50, A61L 27/54, A61L 31/10, A61L 31/14, A61L 31/16

(54) **COMPOSITION FOR SURFACE MODIFICATION OF MEDICAL IMPLANT AND MEDICAL IMPLANT SURFACE-MODIFIED THEREBY**
ZUSAMMENSETZUNG ZUR OBERFLÄCHENMODIFIZIERUNG VON MEDIZINISCHEN IMPLANTATEN UND DAMIT OBERFLÄCHENMODIFIZIERTES MEDIZINISCHES IMPLANTAT
COMPOSITION POUR LA MODIFICATION DE SURFACE D'UN IMPLANT MÉDICAL ET IMPLANT MÉDICAL MODIFIÉ EN SURFACE PAR CELLE-CI

(30) Priority: 19.10.2017 KR 20170135862
(43) Date of publication of application: 26.08.2020
(73) Proprietor: Seoul National University Hospital, Seoul 03080 (KR)
(72) Inventor: HEO, Chan Yeong, Yongin-si Gyeonggi-do 16850 (KR); PARK, Han Soo, Seongnam-si Gyeonggi-do 13525 (KR); KIM, Byung Hwi, Yongin-si Gyeonggi-do 16968 (KR); SHIN, Byung Ho, Yongin-si Gyeonggi-do 16853 (KR); SHIM, Jung Hee, Seoul 06003 (KR); BIRAJDAR, Mallinath S, Seoul 06974 (KR); CHO, Hyun Joo, Ansan-si Gyeonggi-do 15538 (KR); SUTTHIWANJAMPA, Chanutchamon, Seoul 06974 (KR)
(74) Representative: Lavoix
(86) International application number: PCT/KR2018/008100
(87) International publication number: WO 2019/078454

(56) References cited:
- WO-A1-01/27662
- WO-A2-02/070022
- KR-A- 20150 061 136
- US-A- 5 866 113
- US-A1- 2017 065 749
- CHO, H.: "Development and Characterization of Functional PDMS Modified with Itaconic Acid for Implantable Medical Instrument and Tissue Engineering", MASTER' S THESIS, August 2017 (2017-08-01), pages 1 - 59, XP009520860

## Description

### TECHNICAL FIELD

The disclosure relates to a composition for surface modification of a medical implant and a medical implant surface-modified thereby.

### BACKGROUND ART

Medical implants such as implants, dental implants, stents, screws and bone replacements are inserted for the purpose of treatment or plastic surgery. However, being inserted into the body, the medical implants are recognized as foreign substances in the body, and a clotting mechanism is activated, or foreign body reaction is generated to prevent bleeding and blood loss. In addition, since medical implants make permanent contact with biological tissues, the surface biocompatibility and *in vivo* affinity thereof are required.

Accordingly, technique for modifying the surface of medical implants and effective methods for fixing bioactive materials are required.

Meanwhile, recently, it has been reported that itaconic acid inhibits the decomposition enzyme of isocitric acid, which is an important enzyme of the energy metabolic pathway of microorganisms, thereby contributing to the antibacterial activity of macrophages.

CHO, H.: "Development and Characterization of Functional PDMS Modified with Itaconic Acid for Implantable Medical Instrument and Tissue Engineering",Master' s Thesis, August 2017 (2017-08), pages 1-59, discloses chemical modifications of PDMS medical implants by silanization followed by reaction with itaconic acid to reduce biofilm formation, inflammation and capsular contraction of breast implants. The silane functionalities comprise amino groups to form an amide binding with itaconic acid, the contact angle being 73 or 35 degrees, depending on the concentration of itaconic acid.

Accordingly, the inventors of the disclosure confirmed the fibrosis inhibition and inflammatory response inhibition at the implantation site of medical implants by using itaconic acid and completed the inventive concept.

### DESCRIPTION OF EMBODIMENTS

### TECHNICAL PROBLEM

An aspect is to provide a medical implant as defined in claim 1.

Another aspect is to provide a method of manufacturing a medical implant, as defined in claim 6.

### SOLUTION TO PROBLEM

In the disclosure, the itaconic acid may be represented by Formula 1 below.

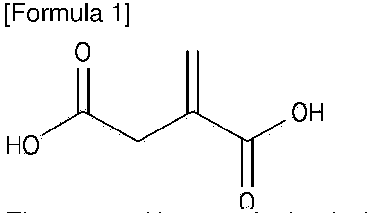

In the disclosure, the term "gelatin" may mean a protein obtained by partial hydrolysis of collagen which is the main protein component of connective tissues such as bones, cartilages, skins, etc., of animals. The gelatin may include gelatin derivatives in addition to pure gelatin. For example, the gelatin derivative may include at least one of a phthalated gelatin, an esterified gelatin, an amidated gelatin, or a formylated gelatin. Relating to gelatin, the kind (source) thereof is not specifically limited, and various kinds of gelatin derived from, for example, mammals and fishes, for example, cow bones, cow skins, swine bones, swine skins, etc., may be used. In addition, the gelatin may have a molecular weight from about 10,000 to about 30,000.

In the disclosure, the term "surface modification" may mean the change of the chemical structure and physical structure of the surface of particles.

The itaconic acid and gelatin may be a nanoparticle type or a polymer type.

At least a portion of the surface of the medical implant may comprise an amino group. Particularly, the surface of the medical implant is silanized for the binding with itaconic acid. An aminosilane compound for silanization may be 3-aminopropyltriethoxysilane (APTES), (3-aminopropyl)-tetraethylorthosilicate, [3-(2-aminoethylamino)propyl]trimethoxysilane, 3-(2-(2-aminoethylamino)ethylamino)propyltrimethoxysilane, or combinations thereof.

The medical implant may be a biocompatible material. Examples of the biocompatible material may include one or more materials selected from the group consisting of gold (Au), silver (Ag), platinum (Pt), palladium (Pd), copper (Cu), steel, tantalum (Ta), magnesium (Mg), nickel (Ni), chromium (Cr), iron (Fe), a nitinol alloy (NiTi), a cobalt-chromium alloy (CoCr) gallium arsenic (GaAs), titanium (Ti), polylactic acid, poly(glycolic acid) (PGA), poly(lactic-co-glycolic acid) (PLGA), poly-ε-(caprolactone), polyanhydride, polyorthoester, polyvinyl alcohol, polyethylene glycol, polyurethane, polyacrylic acid, poly-N-isopropylacrylamide, poly(ethylene oxide)-poly(propylene oxide)-poly(ethylene oxide), polytetrafluoroethylene, polycarbonate, polyurethane, nitrocellulose, polystyrene, polyethylene, polyethylene terephthalate (PET), polydimethylsiloxane (PDMS), polyether ether ketone (PEEK), silicon oxide (SiO₂), titanium oxide (TiO₂), aluminum oxide (Al₂O₃), niobium oxide (Nb₂O₅), silicon, silicone rubber, and glass.

In addition, the medical implant may be selected from the group consisting of breast implants, spinal implants, dental implants, cardiovascular implants, cardiac implants, stents, artificial joints, artificial head bones, and cell culture mediums.

In a particular embodiment, the surface modified medical implant may have a water contact angle from about 20° to about 90°, from about 25° to about 85°, from about 25° to about 80°, from about 25° to about 70°, from about 30° to about 60°, or from about 30° to about 40°.

In another particular embodiment, the surface modified medical implant may have improved fibrosis inhibition or inflammatory response inhibition at an implantation site when compared with that before surface modification.

Another aspect provides a method of surface modifying a medical implant, as defined in claim 6.

In addition, the step of binding itaconic acid may be a step of binding gelatin additionally. This step may include a step of preparing a composite of itaconic acid and gelatin and then, binding, binding itaconic acid and then, binding gelatin, or binding gelatin and then, combining itaconic acid.

### ADVANTAGEOUS EFFECTS OF DISCLOSURE

According to the invention itaconic acid is bound to the surface of the medical implant in high binding stability, and effects showing activity on fibrosis inhibition and inflammatory response inhibition at an implantation site may be achieved.

### BRIEF DESCRIPTION OF DRAWINGS

FIG. 1 is a diagram schematically showing the manufacturing process of a medical implant surface-modified with itaconic acid according to an embodiment.
FIG. 2 is a diagram schematically showing the manufacturing process of a medical implant surface-modified with itaconic acid and gelatin according to an embodiment.
FIG. 3 is a graph showing the contact angles of medical implants surface-modified with itaconic acid and/or gelatin according to an embodiment.
FIG. 4 shows scanning electron microscopy micrographs of medical implants surface-modified with itaconic acid and/or gelatin according to an embodiment.
FIG. 5 is a graph showing the ATR-FTIR analysis results of implants surface-modified with itaconic acid and/or gelatin according to an embodiment.
FIG. 6 is a graph confirming the absorption degree of protein of implants surface-modified with itaconic acid and/or gelatin according to an embodiment.
FIG. 7 is a graph showing the thermal stability of implants surface-modified with itaconic acid and/or gelatin according to an embodiment.
FIG. 8 shows photomicrographs observing the cell culture results of implants surface-modified with itaconic acid and/or gelatin according to an embodiment.
FIG. 9 shows graphs on the analysis results of cell proliferation rates at implants surface-modified with itaconic acid and/or gelatin according to an embodiment.
FIG. 10 shows photographs on the analysis results of cell viability at implants surface-modified with itaconic acid and/or gelatin according to an embodiment.
FIG. 11 shows microscopic photographs analyzing cell attachment patterns at implants surface-modified with itaconic acid and/or gelatin according to an embodiment.

### MODE OF DISCLOSURE

Hereinafter, the present disclosure will be explained in more detail referring to embodiments. However, the embodiments are illustrated for explaining the present disclosure, and the scope of the present disclosure is not limited thereto. In the examples, only the embodiments comprising silanization, binding of itaconic acid and gelatin are according to the claims.

### Example 1 Manufacture of medical implant surface-modified with itaconic acid (IA)

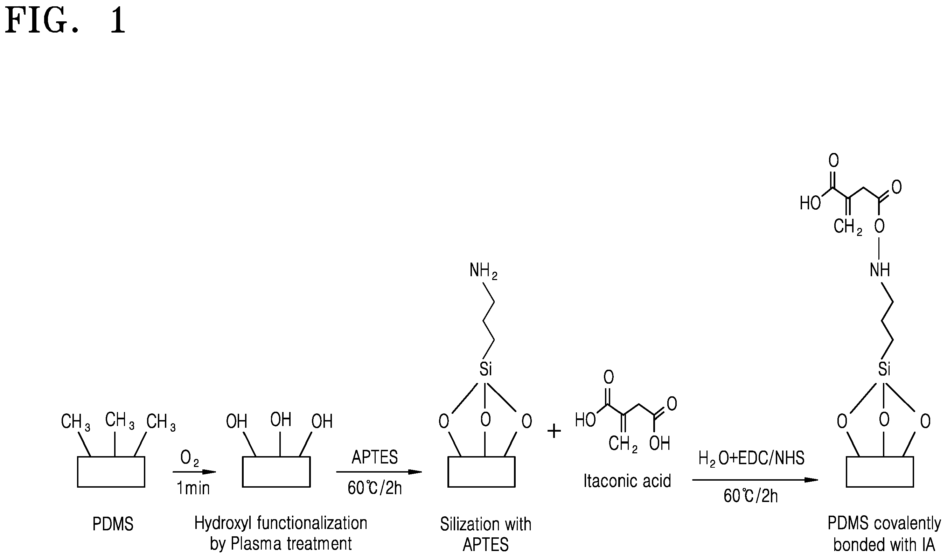

A medical implant surface-modified with itaconic acid was manufactured as shown in FIG. 1.

Particularly, a polydimethylsiloxane (PDMS) substrate (Sylgard^{®} 184 silicone elastomer kit, Dow Corning, USA) was treated with oxygen plasma (CUTE-1B, Femto Science, USA) for 1 minute so as to attach hydroxyl groups (-OH) on the surface of PDMS. After that, the surface of the substrate was silanized using 3-aminopropyl triethoxysilane (APTES) at 60°C for 2 hours. Then, 50 mmol, or 150 mmol of itaconic acid (IA, analytical grade, assay ≥99%, MW: 130.10 g/mol, density: 1.573 g/mL at 25°C (lit.), Sigma-Aldrich, South Korea) was reacted at 60°C for 2 hours in the presence of 1-ethyl-3-(3-dimethylamino)propyl carbodiimide (EDC) and N-hydroxy succinimide (NHS) (EDC/NHS) to bind the itaconic acid with the surface of the PDMS substrate. Then, EDC/NHS residues were removed, and drying was performed for 12 hours to manufacture a medical implant surface-modified with itaconic acid (hereinafter, will be referred to as "IA-PDMS").

### Example 2 Manufacture of medical implant surface-modified with itaconic acid and gelatin.

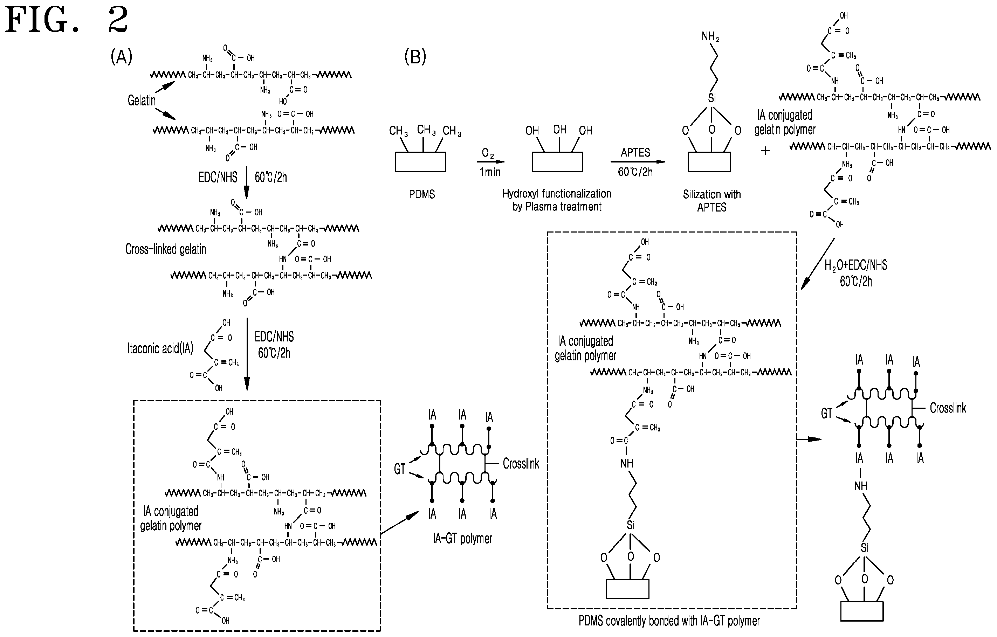

A medical implant surface-modified with itaconic acid and gelatin (IA-GT) was manufactured as shown in FIG. 2.

Particularly, in order to prepare a polymer of itaconic acid and gelatin, a gelatin type B (derived from cow skin, Bloon 75) powder was mixed with DPBS and dissolved at 135°C to 140°C for 16 hours. Then, with the dissolved gelatin type B, 50 mmol of EDC and 50 mmol of NHS solutions were mixed, and 100 mmol of itaconic acid was put and reacted for 2 hours. Additionally, DPBS was added to the mixture, and the reaction with gelatin and itaconic acid was performed at 60°C for 30 minutes and then finished. The final mixture was dialyzed through a cellulose membrane (MWCO 6-8kD), and unreacted itaconic acid, EDC/NHS, and salts were removed. After that, freeze drying was performed at -80°C for 48 hours to obtain an IA-GT powder.

The IA-GT powder thus obtained was binded with the surface of a PDMS substrate as in Example 1 to manufacture a medical implant surface-modified with itaconic acid and gelatin (hereinafter, will be referred to as "IA-GTpoly-PDMS"). Briefly, a surface modified PDMS was manufactured by the same method as in Example 1 except for using DW + EDC 10 mmol + NHS 10 mmol to manufacture the surface modified PDMS of IA-GTpoly-0.25 wt%, and using DW + EDC 20 mmol + NHS 20 mmol to manufacture the surface modified PDMS of IA-GTpoly-0.50 wt%.

### Experimental Example 1 Analysis on properties of implant surface-modified with itaconic acid and/or gelatin

### 1.1. Hydrophilicity analysis

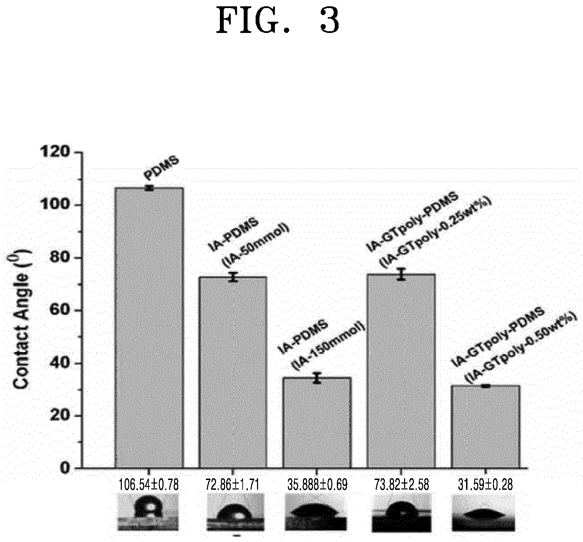

On the surface of the implants manufactured in Examples 1 and 2, 4 µl of distilled water was dropped, and contact angles were measured at room temperature using a laboratory-made contact angle goniometer with a charge-coupled device camera (IMT 3, IMT Solutions), and the results are shown in FIG. 3.

FIG. 3 is a graph showing the contact angles of implants surface-modified with itaconic acid and/or gelatin according to an embodiment.

As shown in shown FIG. 3, it could be found that the PDMSs surface-modified with itaconic acid and/or gelatin had smaller contact angles when compared with the conventional PDMS (control group).

### 1.2. Surface morphology analysis

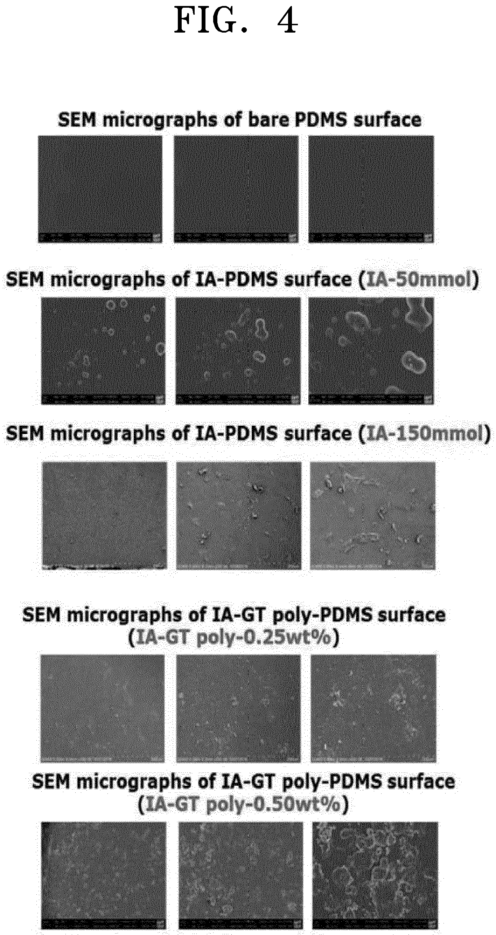

The surfaces of the implants manufactured in Examples 1 and 2 were observed by a scanning electron microscope (SEM) (SEM, S-3400N, Hitachi, Tokyo, Japan), and the results are shown in FIG. 4.

FIG. 4 shows scanning electron microscopy images showing the contact angles of implants surface-modified with itaconic acid and/or gelatin according to an embodiment.

As shown in FIG. 4, it could be confirmed that white modification layers were observed on the surface of PDMSs.

### 1.3. Surface chemical bond analysis

In order to confirm the chemical bond at the surface of the implant, attenuated total reflection (ATR)-FTIR analysis was performed.

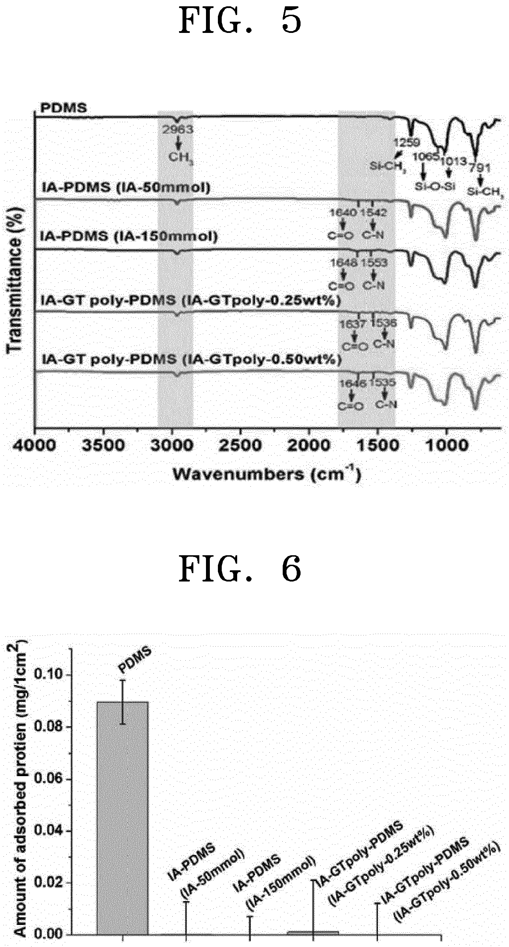

Particularly, with respect to the implants manufactured in Examples 1 and 2, each spectrum at room temperature was recorded from 400 cm⁻¹ to 4000 cm⁻¹ with a resolution of 4 cm⁻¹ using a FT-IR Spectroscopy (Frontier spectrophotometer equipped with and attenuated total reflection (ATR-FTIR) module (Nicolet 6700, Thermo Scientific, USA), the scanning was performed 200 times and averaged, and the results are shown in FIG. 5.

FIG. 5 is a graph showing the ATR-FTIR analysis results of implants surface-modified with itaconic acid and/or gelatin according to an embodiment.

As shown in FIG. 5, the peaks of carbonyl groups (1,640 cm⁻¹ for IA-50 mmol, 1,648 cm⁻¹ for IA-150 mmol, 1,637 cm⁻¹ for IA-GTpoly-0.25 wt% & 1,646 cm⁻¹ for IA-GTpoly-0.50 wt%) were observed, and through the results, the binding of IA with PDMS was confirmed. In addition, through the observation of the peaks of nitrile groups (1,542 cm⁻¹ for IA-50 mmol, 1,553 cm⁻¹ for IA-150 mmol, 1,536 cm⁻¹ for IA-GTpoly-0.25 wt% & 1,535 cm⁻¹ for IA-GTpoly-0.50 wt%), amide bonds were confirmed.

### 1.4. Protein absorbing capacity analysis

The protein absorbing capacity of the surfaces of the implants manufactured in Examples 1 and 2 was analyzed.

Particularly, bovine serum albumin (BSA) (4.5 mg/mL) was dissolved in DPBS. Separately, the surfaces of IA-PDMS and IA-GTpoly-PDMS (size: 1 cm²) were cultured on a hot stirring plate in a protein solution while stirring constantly for 1 hour at 37°C in 200 rpm conditions. After washing with clean DPBS twice, BSA protein - cultured bare, IA-conjugated and IA-GTpoly conjugated PDMS surfaces (triple) were transported to a 24-well plate containing DW and an operation reagent mixture. After culturing the well plate at 37°C for 30 minutes, the absorbance value of each sample was analyzed. The amount of absorbed protein was quantified using Micro BCA^{™} protein analysis kit according to the instruction of a manufacturer. The absorbance value was measured using a microplate spectrophotometer with a fixed wavelength of 570 nm. All values were represented by average ± standard deviation, and the results are shown in FIG. 6.

FIG. 6 is a graph confirming the absorption degree of protein of implants surface-modified with itaconic acid and/or gelatin according to an embodiment.

As shown in FIG. 6, it could be confirmed that strong inhibition of protein absorption was observed for the implants surface-modified with itaconic acid and/or gelatin according to an embodiment when compared with the control group.

### 1.5. Binding stability analysis

In order to analyze the binding stability of itaconic acid and/or gelatin, heat treatment was performed with respect to the surface of the implants manufactured in Examples 1 and 2.

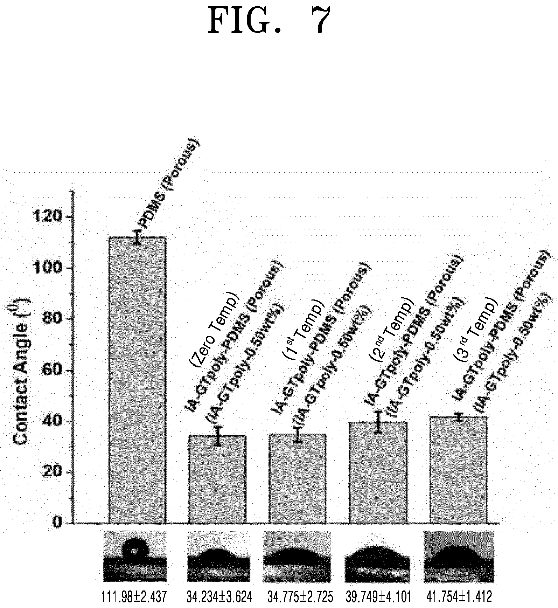

Particularly, IA-GTpoly-PDMS (IA-GTpoly-0.50 wt%) having a size of 2 cm² was used as a specimen for heat treatment, and the heat treatment was performed three times in total at 120°C for 1 hour. After that, contact angles were measured by the same method as in Experimental Example 1.1, and the results are shown in FIG. 7.

FIG. 7 is a graph showing the thermal stability of implants surface-modified with itaconic acid and/or gelatin according to an embodiment.

As shown in FIG. 7, it could be confirmed that after the heat treatment three times, each contact angle was gradually increased, but the change of the binding stability of itaconic acid and/or gelatin was not observed.

### Experimental Example 2 Activity analysis of implant surface-modified with itaconic acid and/or gelatin

### 2.1. In vitro analysis of cell culture ability

### 2.1.1. Analysis of cell proliferation rate and viability

In order to analyze the cell proliferation rate and viability in the implants manufactured in Examples 1 and 2, NIH3T3 mouse fibroblast lines were used.

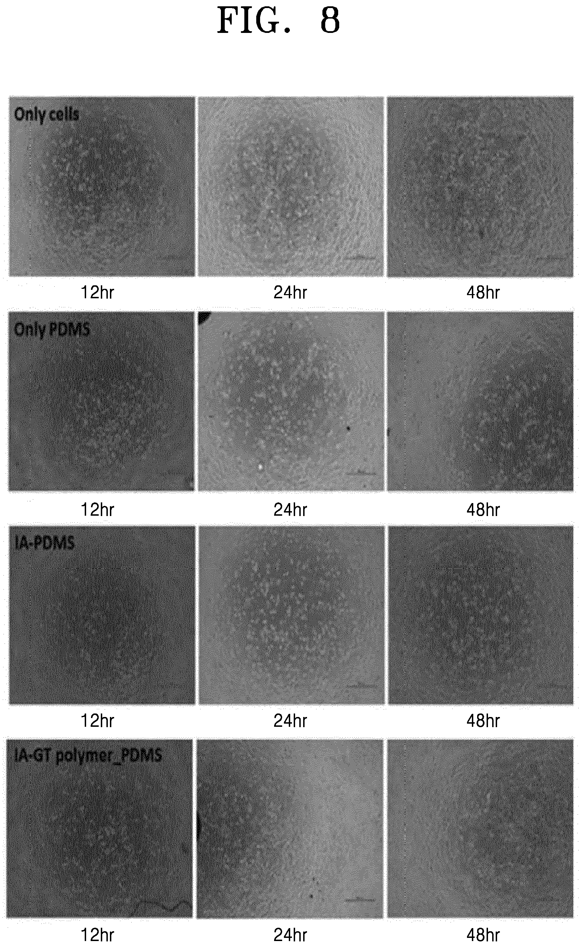

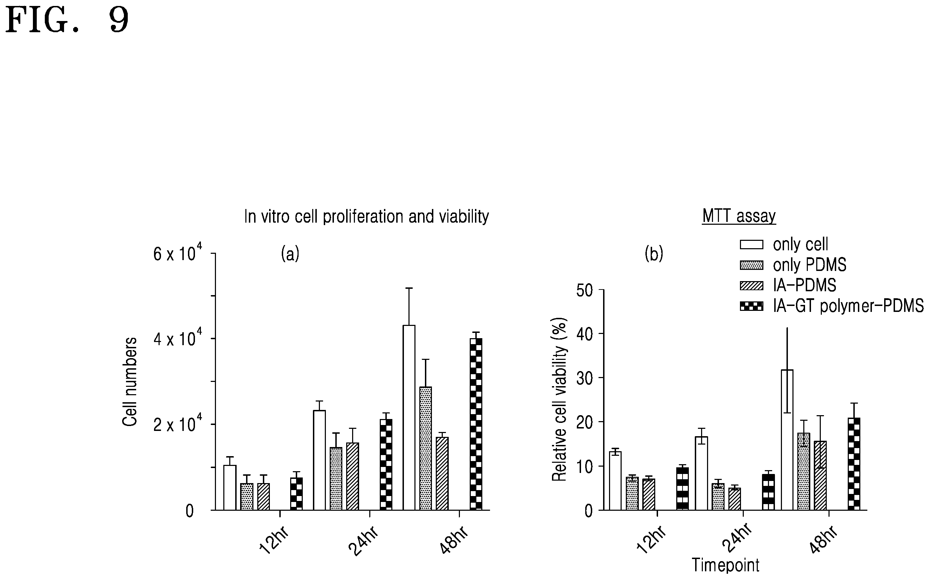

Particularly, the fibroblast lines were cultured at the surface of PDMS having a size of 1 cm x 1 cm with cells of 20,000 cell/ml at 37°C. After initiating the culture, the cell proliferation rates and viability at 12 hour, 24 hour and 48 hour were analyzed. The cell proliferation rate was observed by an optical microscope (Euromex IF-Series, Netherlands), by treating cells with trypsin (GE Healthcare Life Sciences HyClone Laboratories, USA.) and counting the number of cells, or by counting the number of cells through an MTT assay (Cell Biolabs, INC, USA). The observation results by the optical microscope are shown in FIG. 8, and the counting results of cells after treating with trypsin and the MTT assay results are shown in FIG. 9.

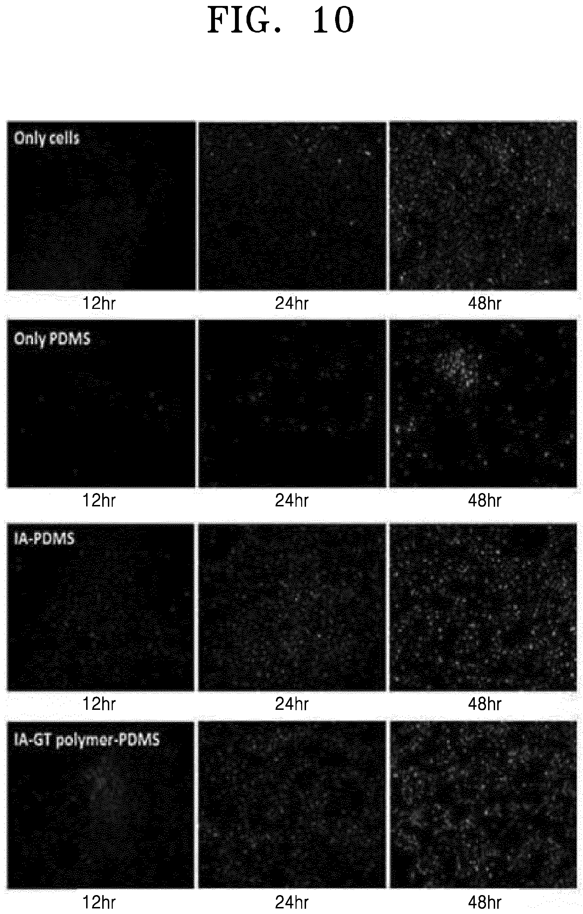

In addition, the cell viability was observed through a Live/Dead analysis method (L-3224, Invitrogen, LIVE/DEAD^{®} Viability/Cytotoxicity Kit, for mammalian cells (Molecular Probes^{®})). Particularly, in order to prepare a Live/Dead analysis solution, 10 ml of PBS was added to a 15 ml tube, and 2 mM EthD-1 and 4 mM calcein AM were added in 20 µl and 5 µl, respectively. Then, the tube was wrapped with an aluminum foil, well mixed through pipetting, and stored under refrigeration. In addition, the cultured cells were put in PBS and washed for 20 minutes, PBS was removed, and the Live/Dead analysis solution thus prepared was added to a cell specimen. After that, culturing was performed in an incubator for 30 minutes, photographs were taken using a fluorescence and confocal microscope, and the results are shown in FIG. 10.

FIG. 8 shows optical microscope photographs observing the cell culture results of implants surface-modified with itaconic acid and/or gelatin according to an embodiment.

FIG. 9 shows graphs showing the analysis results of cell proliferation rates at implants surface-modified with itaconic acid and/or gelatin according to an embodiment.

FIG. 10 shows photographs showing the analysis results of cell viability at implants surface-modified with itaconic acid and/or gelatin according to an embodiment.

As shown in FIG. 8, it could be found that the cell culture was conducted well at the implants surface-modified with itaconic and/or gelatin according to an embodiment.

In addition, as shown in FIG. 9, the implants surface-modified with itaconic acid and/or gelatin according to an embodiment showed similar or increased cell proliferation rates when compared with the control group.

In addition, as shown in FIG. 10, the implants surface-modified with itaconic acid and/or gelatin according to an embodiment showed higher cell viability when compared with the surface unmodified PDMS of the control group.

### 2.1.2. Analysis of cell attachment pattern

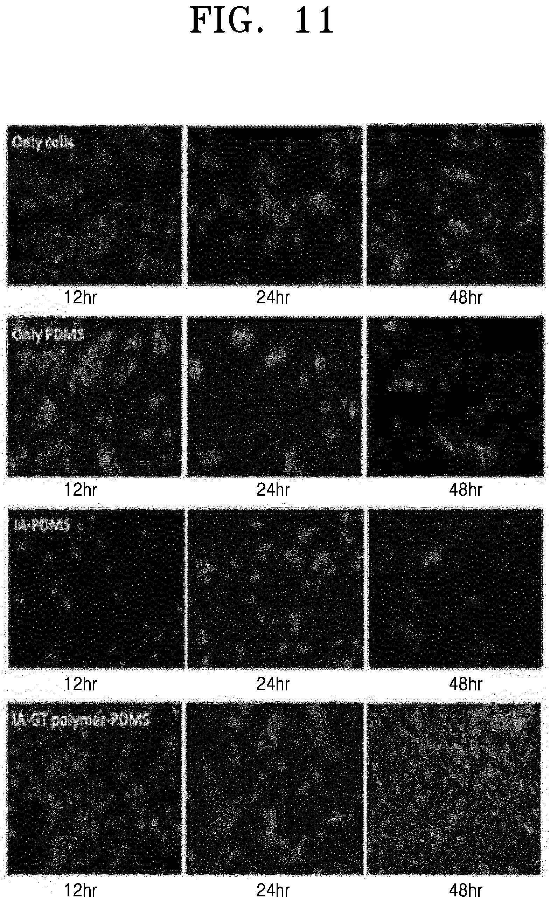

In order to analyze cell attachment pattern, the cells were stained with a Rhodamin/DAPI staining (Invitrogen, ThermoFisher Scientific, USA). This was observed with a fluorescence microscope (CKX-41, OLYMPUS, Japan), and the results are shown in FIG. 11.

FIG. 11 shows microscopic photographs analyzing cell attachment patterns at implants surface-modified with itaconic acid and/or gelatin according to an embodiment.

As shown in FIG. 11, the cells were spread better at the implants surface-modified with itaconic acid and/or gelatin according to an embodiment.

### 2.2. In vivo activity analysis

### 2.2.1. Analysis of fibrosis at implantation site

The implants manufactured in Examples 1 and 2 were transplanted to animal models, and the fibrosis states at implantation sites were observed.

First, four 8-week Sprague-Daley rats (Young Bio, KOREA) were used for each group as the animal models. Animal experiment was conducted after final approval from Institutional Animal Care and Use Committee (IACAU) by Seoul National University Hospital in Bundang (approval number: BA1608-206/045-01). Experiment was conducted by dividing into five groups as follows. In order to prevent inflammation and infection at a wounded area during transplanting an implant, disinfection was conducted using betadine. The disinfection was performed twice before and after surgical procedure. For transplantation, the back was incised by 2 cm, a subcutaneous pocket was formed, and implants of each group were transplanted in the pocket. After the transplantation, the wounded area was sutured using Nylon 4/0 (Ethicon), the wounded area was finally disinfected using betadine, and a gauze dressing was applied.

Five groups of implants below were all transplanted in one rat, the rat was euthanized after 2, 4 and 8 weeks, and tissues at an implantation site were obtained.
Group 1 (control group): untreated PDMS
Group 2 (IA 50 mmol (low) PDMS): PDMS surface treated with IA having a low concentration
Group 3 (IA 150 mmol (high) PDMS): PDMS surface treated with IA having a high concentration
Group 4 (IA-GT 0.25% (low) PDMS): PDMS surface treated with IA chemically bonded to gelatin, having a low concentration
Group 5 (IA-GT 0.50% (high) PDMS): PDMS surface treated with IA chemically bonded to gelatin, having a high concentration

In order to evaluate a fibrosis degree, i) capsule thickness, ii) number of fibroblast and iii) number of myofibroblast, were quantified.

Particularly, capsule thickness was analyzed through staining with Hematoxylin & Eosin. The capsule thickness was measured with respect to collagen and tissue layers accumulated on the bottom part of subcutaneous muscles, the analysis was performed by measuring a portion where the thinnest image phase capsule was formed, and the results are shown in Table 1.

**[Table 1]**

| Capsule thickness results (unit: µm) | | | | | |
|---|---|---|---|---|---|
| Week | Control | IA_Low | IA_High | IA_GT Low | IA_GT High |
| 2 | 813 ± 260.9 | 514.8 ± 302.3 | 487.9 ± 45.8 | 403.3 ± 145 | 323.2 ± 158.5 |
| 4 | 1060.6 ± 83.7 | 527.9 ± 110.6 | 621.7 ± 70 | 506.6 ± 73.1 | 544.8 ± 60.5 |
| 8 | 1426.1 ± 250.6 | 789.1 ± 284.9 | 786.7 ± 210.5 | 774 ± 136.9 | 528.6 ± 183.3 |

As shown in Table 1, IA-PDMS and IA-GT-PDMS groups were confirmed to show decreased capsule thicknesses in all cases after 2, 4 and 8 weeks when compared with the control group, and accordingly, it could be found that the fibrosis was reduced by IA. Particularly, the thinnest capsule thickness was observed in IA-GT-PDMS high, and this means that the capsule thickness of IA-GT high PDMS was reduced to about 63% when compared with the control group (IA-GT high: 528 µm (37%) against control group: 1426 µm (100%)).

In order to evaluate the number of fibroblast in tissues, immunostaining was performed, only fibroblast was specifically stained using an antibody (ab92546, Abcam, CA, USA) binded with Vimentin which is a specific factor of fibroblast, and only cells expressing fluorescence were selectively evaluated to confirm the number of cells. Particularly, for the progress of the experiment, the biopsy of tissues was performed after a certain time period, and the biopsied tissues were manufactured into slides through a paraffin embedded technique. After the pre-treatment of the slide (Deparaffine, Rehydration), an immune factor bonding to the fibroblast specific factor was used, color expressed by the bonding of the specific factor and DAPI where staining of a cell nucleus occurs were selectively counted and analyzed per week, and the results are shown in Table 2.

**[Table 2]**

| Fibroblast number (unit: n) | | | | | |
|---|---|---|---|---|---|
| Week | Control | IA_Low | IA_High | IA_GT Low | IA_GT High |
| 2 | 36.6 ± 13.41 | 32 ± 4.73 | 24.17 ± 4.88 | 28.83 ± 5.81 | 27.17 ± 6.15 |
| 4 | 27.5 ± 11.48 | 31.67 ± 10.93 | 23.67 ± 7.45 | 29.67 ± 5.16 | 27.17 ± 5.67 |
| 8 | 39 ± 11.83 | 30.17 ± 13.38 | 21 ± 5.52 | 23.8 ± 7.66 | 20.75 ± 5.06 |

As shown in Table 2, somewhat lower number of fibroblasts was confirmed in all groups until 2 weeks and 4 weeks from transplantation when compared with the control group, but the difference was insignificant. However, in groups after 8 weeks from the transplantation, smaller fibroblasts were observed in IA high and IA-GT high groups when compared with the control group (p<0.05). This corresponds to an effect of reducing the collagen density of IA-GT high PDMS to about 50% when compared with the control group (IA high: 21 (53.8%) / IA-GT high: 20.75 (53.2%) against control group: 39 (100%)).

In order to evaluate the number of myofibroblasts in tissues, immunostaining was performed, only myofibroblasts were specifically stained using an antibody binded with alpha-SMA which is a specific factor of myofibroblast, and only cells expressing fluorescence were selectively evaluated to confirm the number of cells. Particularly, for the progress of the experiment, the biopsy of tissues was performed after a certain time period, and the biopsied tissues were manufactured into slides through a paraffin embedded technique. After the pre-treatment of the slide (Deparaffine, Rehydration), an immune factor bonding to a myofibroblast specific factor was used, color expressed by the bonding of the specific factor and DAPI where staining of a cell nucleus occurs were selectively counted and analyzed per week, and the results are shown in Table 3.

**[Table 3]**

| Myofibroblast number (unit: n) | | | | | |
|---|---|---|---|---|---|
| Week | Control | IA_Low | IA_High | IA_GT Low | IA_GT High |
| 2 | 46 ± 6.03 | 41.17 ± 4.12 | 29.5 ± 5.17 | 39.5 ± 5.68 | 34.33 ± 7.5 |
| 4 | 62.5 ± 14.03 | 49.5 ± 12 | 32 ± 10.53 | 37.17 ± 8.21 | 41.17 ± 1.94 |
| 8 | 40 ± 11.73 | 37.5 ± 7.79 | 29.83 ± 10 | 33 ± 6.72 | 27.67 ± 8.78 |

As shown in Table 3, the significant decrease of myofibroblasts was observed in IA high group after 2 weeks from transplantation. In addition, after 4 weeks, the significant decrease of myofibroblasts was confirmed in all experimental groups (IA high, IA-GT low, IA-GT high) excluding the IA low group when compared with the control group (p<0.05). In the groups after 8 weeks, the decrease of the myofibroblast number was observed in IA high and IA-GT high groups when compared with the control group. This corresponds to an effect of decreasing the collagen density of IA-GT high PDMS to about 50% when compared with the control group (IA high: 21 (53.8%) / IA-GT high: 20.75 (53.2%) against control group: 39 (100%)).

### 2.2.2. Inflammatory response analysis

In order to analyze inflammatory response at an implantation site, biopsied tissues were stained using Hematoxylin & Eosin. Then, only polynuclear cells (inflammatory mediated cells) in the capsule formation area of stained tissues were selected and quantified using an image software. Particularly, the staining with Hematoxylin & Eosin was performed for biopsied tissues, and only cells considered to be polynuclear cells after the staining of the tissues were specifically selected and counted. Scores were configured based on the counted specific polynuclear cells, inflammatory response score in each group was analyzed according to the range of the configuration, and the results are shown in Table 4.

**[Table 4]**

| Inflammatory response score (unit: score) | | | | | |
|---|---|---|---|---|---|
| Week | Control | IA_Low | IA_High | IA_GT Low | IA_GT High |
| 2 | 2.48 ± 0.44 | 1.96 ± 0.15 | 1.82 ± 0.29 | 1.58 ± 0.38 | 1.3 ± 0.31 |
| 4 | 1.98 ± 0.43 | 1.54 ± 0.27 | 1.38 ± 0.27 | 1.37 ± 0.53 | 1.17 ± 0.43 |
| 8 | 1.35 ± 0.14 | 1.42 ± 0.4 | 1.03 ± 0.24 | 1.05 ± 0.33 | 1 ± 0.3 |

As shown in Table 4, it could be found that the inflammatory response was significantly decreased in all experimental groups when compared with the control group.

From the results above, it could be found that an implant surface-modified with itaconic acid and gelatin according to an embodiment reduces fibrosis or inflammatory response at an implantation site when compared with a control group and accordingly, the implant could be useful as a medical implant.

## Claims

1. A medical implant, wherein at least a portion of a surface of the implant is bound with itaconic acid for surface modification,
wherein the at least a portion of the surface is silanized for binding with the itaconic acid, and
wherein gelatin is crosslinked and bound with the surface of the implant through the itaconic acid.

2. The implant according to claim 1, wherein the medical implant comprises one or more materials selected from the group consisting of gold (Au), silver (Ag), platinum (Pt), palladium (Pd), copper (Cu), steel, tantalum (Ta), magnesium (Mg), nickel (Ni), chromium (Cr), iron (Fe), a nitinol alloy (NiTi), a cobalt-chromium alloy (CoCr) gallium arsenic (GaAs), titanium (Ti), polylactic acid, poly(glycolic acid) (PGA), poly(lactic-co-glycolic acid) (PLGA), poly-ε-(caprolactone), polyanhydride, polyorthoester, polyvinyl alcohol, polyethylene glycol, polyurethane, polyacrylic acid, poly-N-isopropylacrylamide, poly(ethylene oxide)-poly(propylene oxide)-poly(ethylene oxide), polytetrafluoroethylene, polycarbonate, polyurethane, nitrocellulose, polystyrene, polyethylene, polyethylene terephthalate (PET), polydimethylsiloxane (PDMS), polyether ether ketone (PEEK), silicon oxide (SiO₂), titanium oxide (TiO₂), aluminum oxide (Al2O₃), niobium oxide (Nb₂O₅), silicon, silicone rubber, and glass.

3. The implant according to claim 1, wherein a water contact angle is from 20° to 90°.

4. The implant according to claim 1, wherein the medical implant is selected from the group consisting of a breast implant, a spinal implant, a dental implant, a cardiovascular implant, a cardiac implant, a stent, an artificial joint, an artificial head bone, and a cell culture medium.

5. The implant according to claim 1, wherein fibrosis inhibition or inflammatory response inhibition at an implantation site is increased when compared with that before surface modification.

6. A method of surface modifying a medical implant, the method comprising:
a step of functionalizing at least a portion of a surface of the medical implant; and
a step of binding itaconic acid with the surface of the functionalized medical implant, wherein the at least a portion of the surface is silanized for the binding with the itaconic acid,
wherein gelatin is additionally bound in the step of binding itaconic acid.

## Patentansprüche

1. Medizinisches Implantat, wobei mindestens ein Abschnitt einer Oberfläche des Implantats zur Oberflächenmodifikation mit Itaconsäure gebunden ist,
wobei der mindestens eine Abschnitt der Oberfläche zur Bindung mit der Itaconsäure silanisiert ist, und
wobei Gelatine durch die Itaconsäure vernetzt und an die Oberfläche des Implantats gebunden ist.

2. Implantat nach Anspruch 1, wobei das medizinische Implantat ein oder mehrere Materialien umfasst, die ausgewählt sind aus der Gruppe, bestehend aus Gold (Au), Silber (Ag), Platin (Pt), Palladium (Pd), Kupfer (Cu), Stahl, Tantal (Ta), Magnesium (Mg), Nickel (Ni), Chrom (Cr), Eisen (Fe), einer Nitinol-Legierung (NiTi), einer Kobalt-Chrom-Legierung (CoCr), Galliumarsen (GaAs), Titan (Ti), Polymilchsäure, Poly(glykolsäure) (PGA), Poly(milch-co-glykolsäure) (PLGA), Poly-ε-(caprolacton), Polyanhydrid, Polyorthoester, Polyvinylalkohol, Polyethylenglykol, Polyurethan, Polyacrylsäure, Poly-N-isopropylacrylamid, Poly(ethylenoxid)-poly(propylenoxid)-poly(ethylenoxid), Polytetrafluorethylen, Polycarbonat, Polyurethan, Nitrocellulose, Polystyrol, Polyethylen, Polyethylenterephthalat (PET), Polydimethylsiloxan (PDMS), Polyetheretherketon (PEEK), Siliziumoxid (SiO₂), Titanoxid (TiO₂), Aluminiumoxid (Al2O₃), Nioboxid (Nb₂O₅), Silizium, Silikonkautschuk und Glas.

3. Implantat nach Anspruch 1, wobei der Wasserkontaktwinkel zwischen 20° und 90° ist.

4. Implantat nach Anspruch 1, wobei das medizinische Implantat ausgewählt ist aus der Gruppe, bestehend aus einem Brustimplantat, einem Wirbelsäulenimplantat, einem Zahnimplantat, einem kardiovaskulären Implantat, einem Herzimplantat, einem Stent, einem künstlichen Gelenk, einem künstlichen Kopfknochen und einem Zellkulturmedium.

5. Implantat nach Anspruch 1, wobei Fibrosehemmung oder Hemmung einer Entzündungsreaktion an einer Implantationsstelle im Vergleich zu jener vor der Oberflächenmodifikation erhöht ist.

6. Verfahren zur Oberflächenmodifizierung eines medizinischen Implantats, das Verfahren umfassend:
einen Schritt eines Funktionalisierens mindestens eines Abschnitts einer Oberfläche des medizinischen Implantats; und
einen Schritt eines Bindens von Itaconsäure an die Oberfläche des funktionalisierten medizinischen Implantats, wobei der mindestens eine Abschnitt der Oberfläche zur Bindung mit der Itaconsäure silanisiert ist,
wobei in dem Schritt des Bindens von Itaconsäure zusätzlich Gelatine gebunden wird.

## Revendications

1. Implant médical, dans lequel au moins une partie d'une surface de l'implant est liée à de l'acide itaconique pour modifier la surface,
dans lequel l'au moins une partie de la surface est silanisée pour être liée à l'acide itaconique, et
dans lequel de la gélatine est réticulée et liée à la surface de l'implant par l'acide itaconique.

2. Implant selon la revendication 1, dans lequel l'implant médical comprend un ou plusieurs matériaux choisis dans le groupe constitué par l'or (Au), l'argent (Ag), le platine (Pt), le palladium (Pd), le cuivre (Cu), l'acier, le tantale (Ta),le magnésium (Mg), le nickel (Ni), le chrome (Cr), le fer (Fe), un alliage de nitinol (NiTi), un alliage cobalt-chrome (CoCr), l'arséniure de gallium (GaAs), le titane (Ti), l'acide polylactique, le poly(acide glycolique) (PGA), le poly(acide lactique-co-glycolique) (PLGA), la poly-ε-(caprolactone), le polyanhydride, le polyorthoester, l'alcool polyvinylique, le polyéthylène glycol, le polyuréthane, l'acide polyacrylique, le poly-N-isopropylacrylamide, le poly(oxyde d'éthylène)-poly(oxyde de propylène)-poly(oxyde d'éthylène), le polytétrafluoroéthylène, le polycarbonate, le polyuréthane, la nitrocellulose, le polystyrène, le polyéthylène, le polyéthylène téréphtalate (PET), le polydiméthylsiloxane (PDMS), le polyéther éther cétone (PEEK), l'oxyde de silicium (SiO₂), l'oxyde de titane (TiO₂), l'oxyde d'aluminium (Al2O₃), l'oxyde de niobium (Nb₂O₅), le silicium, le caoutchouc de silicone et le verre.

3. Implant selon la revendication 1, dans lequel un angle de contact avec l'eau est de 20° à 90°.

4. Implant selon la revendication 1, dans lequel l'implant médical est choisi dans le groupe constitué par un implant mammaire, un implant rachidien, un implant dentaire, un implant cardiovasculaire, un implant cardiaque, une endoprothèse, une articulation artificielle, un os de tête artificiel et un milieu de culture cellulaire.

5. Implant selon la revendication 1, dans lequel l'inhibition de la fibrose ou l'inhibition de la réponse inflammatoire à un site d'implantation est augmentée par rapport à ce qu'elle était avant la modification de surface.

6. Procédé de modification de surface d'un implant médical, le procédé comprenant :
une étape de fonctionnalisation d'au moins une partie d'une surface de l'implant médical ; et
une étape de liaison de l'acide itaconique avec la surface de l'implant médical fonctionnalisée, dans lequel au moins une partie de la surface est silanisée pour la liaison avec l'acide itaconique,
dans lequel la gélatine est également liée lors de l'étape de liaison de l'acide itaconique.
